# EUROPEAN PATENT SPECIFICATION

(11) **EP 2 440 925 B1**
(45) Date of publication and mention of the grant of the patent: **31.07.2013**
(21) Application number: 09779678.3
(22) Date of filing: 08.06.2009
(51) Int. Cl.: G01N 33/52

(54) **METHODS AND KITS FOR DETECTING, DIAGNOSING AND MONITORING DISEASES**
VERFAHREN UND KITS FÜR DEN NACHWEIS, DIE DIAGNOSE UND ÜBERWACHUNG VON ERKRANKUNGEN
PROCÉDÉS ET ENSEMBLES DE DÉTECTION, DIAGNOSTIC ET SURVEILLANCE DE MALADIES

(43) Date of publication of application: 18.04.2012
(73) Proprietor: Protea Biopharma N.v., 1731 Zellik (BE)
(72) Inventor: ROELANT, Christiaan, B-3000 Leuven (BE); DE MEIRLEIR, Kenny, B-2800 Mechelen (BE)
(74) Representative: Paemen, Liesbet R.J.
(86) International application number: PCT/EP2009/057054
(87) International publication number: WO 2010/142322

(56) References cited:
- EP-A2- 0 264 079
- EP-A2- 0 309 882
- DD-A1- 237 223
- GB-A- 2 200 989
- JP-A- 1 213 574
- JP-A- 2005 118 014
- US-A1- 2008 138 793
- VOROTELIAK VICTOR ET AL: "Improved colorimetric determination of urinary thiosulfate to study intermediate sulfur metabolism in humans" CLINICAL CHEMISTRY, AMERICAN ASSOCIATION FOR CLINICAL CHEMISTRY, WASHINGTON, DC, vol. 39, no. 12, 1 January 1993 (1993-01-01), pages 2533-2534, XP008118902 ISSN: 0009-9147
- DATABASE WPI Week 198940 Thomson Scientific, London, GB; AN 1989-289953 XP002568749 & JP 01 213574 A (WAKO PURE CHEM IND LTD) 28 August 1989 (1989-08-28)
- Kage, S.: "Hydrogen sulfide and its metabolite" II.1.2 In: Suzuki, O., Watanabe, K.: "Drugs and Poisons in Humans: A Handbook of Practical Analysis" 2005, Springer Verlag , Berlin Heidelberg , XP8119198 ISBN: 3540222774pages 101-111, the whole document

## Description

### FIELD OF THE INVENTION

The present invention relates to methods an the use of kits for detecting, diagnosing and monitoring diseases based on the detection of metabolites in urine. More particularly, the methods and kits of the present invention are useful in the diagnosis and/or monitoring of diseases such as inflammatory, infectious and chronic (immune) diseases or diseases of unknown origin.

### BACKGROUND

Metabolite measurement in physiological fluids is of ever increasing importance to today's society. Metabolite detection assays find use in a variety of applications, including clinical laboratory testing, home testing, etc., where the results of such testing play a prominent role in diagnosis and management of a variety of disease conditions. In response to this growing importance of metabolite measurement, a variety of metabolite measurement protocols and devices for both clinical and home use have been developed. While a number of signal-producing systems have been developed to date for use in the measurement of a wide variety of different metabolites, there continues to be a need for the further development of such systems.

In particular, there is a need for broad spectrum assays and kits, which allow for easy detection and monitoring of disease states, such as inflammatory and/or chronic immune diseases, to follow-up and monitor efficacy of treatment, to identify diseases which are otherwise "silent" or non-defined illnesses, to alert patients to seek medical advice and/or to actually demonstrate an underlying "physiological" symptom of diseases that are not fully understood or even denied, such as chronic fatigue/ME syndrome, fibromyalgia and the like.

With the exception of glucose testing, pH measurement or specific detection of urine components for e.g. pregnancy or the presence of heavy metals, no "broad spectrum disease" testing assays or kits have been developed up to date.

JP1213574 describes the detection of ascorbic acid in urine of patients using tetrazolium compounds at a pH of 10-11.
JP2005118014 describes the detection of oxidoreductases using tetrazolium salts such as MTT.

### SUMMARY OF THE INVENTION

The invention in its broadest form is defined by independent claims 1 and 11:
1. An in vitro method for detecting or monitoring a disease state in a patient, said method comprising detecting the presence of metabolites in urine by contacting a urine sample of said patient with at least one tetrazolium dye at a pH-value which is higher than the pKa-value of said tetrazolium dye and between 5 and 11 under buffered conditions without adding any additional reducing agents, whereby a reduction of the tetrazolium dye occurs by direct reaction of said metabolites in the urine with said at least one tetrazolium dye and whereby a color change resulting from reduction of the tetrazolium dye is indicative of the increased presence of metabolites in the urine and thus indicative of a disease state in said patient.
11. Use of a kit comprising at least one tetrazolium dye, a buffer with a pH between 5 and 11 which is higher than the pKa value of the tetrazolium dye, a reaction container, a vial for collecting urine, and a pipetting device, wherein said kit does not comprise any additional reducing agents for detecting, diagnosing and/or monitoring a disease state of a patient by detecting increased metabolite production in a urine sample of said patient.

Preferred embodiments are defined by dependent claims 2-10 and 12-14.

### DETAILED DESCRIPTION

Methods described in the present specification for detecting the presence of metabolites in body fluids other than urine and using reducible dyes that are not tetrazolium dyes are for reference only. In the claimed invention, the reducible dye is contacted directly with the urine sample and is reduced by metabolites in the sample.

The present invention is based on the observation that many diseases states cause the aberrant and/or increased production of metabolites that are excreted in the body fluids and that the presence of these metabolites can easily be detected by contacting the body fluid of patients with a reducible dye such as a tetrazolium-based compound.

Accordingly, in one aspect, the present invention provides methods for detecting metabolites in a body fluid by contacting a sample of the body fluid with at least one reducible dye at a pH-value which is higher than the pKa-value of the reducible dye. More particularly the invention provides methods for detecting metabolites in a body fluid by contacting a sample of the body fluid of a patient with at least one reducible dye at a pH-value which is higher than the pKa-value of the reducible dye, whereby a color change resulting from reduction of the reducible dye is indicative of the presence of metabolites in the body fluid of the patient. In particular embodiments, these methods comprise the steps of (i) obtaining a color reagent by dissolving at least one reducible dye compound in a buffer solution, wherein the pH-value of the buffer solution is higher than the pKa- value of the reducible dye, (ii) contacting the sample with the color reagent obtained in step (i), and (iii) determining the color of the color reagent after it has been contacted with the sample. In these methods, the color is indicative of the presence of metabolites in the sample of body fluid used. In further particular embodiments step (iii) of the method comprises comparing the color of the color reagent with a reference.

The methods of the invention are for the analysis of urine.

The methods described herein can be used as diagnostic methods for detecting, diagnosing and/or monitoring aberrant and/or increased metabolite production in a patient. More particularly, the methods described herein can be used for detecting, diagnosing and/or monitoring a disease related to inflammatory, infectious and/or chronic immune disease. Additionally or alternatively the methods can be used to identify and monitor a disease state.

In the methods of the invention described herein, the reducible dye is a tetrazolium-based compound, more specifically at least one reducible dye is used, of which one or all are selected from the group consisting of MTT, XTT, INT, MTS and NBT.

In a further aspect, the present invention provides the use of kits for detecting metabolites in body fluids comprising at least one reducible dye, a reaction container, a vial for collecting body fluid and a pipetting device and a buffer.

More particularly, the invention provides the use of kits for detecting metabolites in a body fluid of a patient, which kits comprise at least one reducible dye, a buffer with a pH which is higher than the pKa value of the reducible dye, a reaction container, a vial for collecting a body fluid, and a pipetting device. Optionally the kit further comprises a reference and/or a package insert. With these components, such kits contain all the necessary reagents and tools for performing the detection, and thus are ideally suited for self-testing or testing at home or in a (non-specialized) medical practice. More particularly, such kits are specifically designed for the detection of metabolites in urine.

The kits of the present invention comprise a reducible dye which is a tetrazolium-based compound.

The kits according to the invention comprise a reaction container which can be a fluid reaction container or a carrier.

In particular embodiments, the kits are provided as diagnostic kits suitable for detecting, diagnosing and/or monitoring increased and/or aberrant metabolite production. More particularly the kits are provided as diagnostic kits suitable for detecting, diagnosing and/or monitoring a disease related to inflammatory, infectious and/or chronic immune disease. More particularly the kits are provided for identifying and monitoring a 'disease state' (more particularly one which can be diagnosed as one of the diseases listed above), which can be prior to identification of the disease state (i.e. to determine/confirm the presence of a physiological problem) or for monitoring purposes after the nature of the disease has been identified (e.g. to determine efficiency of therapeutic regimen).

Accordingly, a further aspect of the present invention is the use of the kits as described herein for detection, diagnosis and/or monitoring aberrant metabolite production, more particularly for the uses described herein.

### DETAILED DESCRIPTION

Methods described in the present specification for detecting the presence of metabolites in body fluids other than urine and using reducible dyes that are not tetrazolium dyes are for reference only. In the claimed invention, the reducible dye is contacted directly with the urine sample and is reduced by metabolites in the sample.

The present invention will be described with respect to particular embodiments but the invention is not limited thereto but only by the claims. Any reference signs in the claims shall not be construed as limiting the scope.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The terms or definitions used herein are provided solely to aid in the understanding of the invention.

A first aspect of the present invention provides in vitro methods for detecting metabolites in a body fluid of a patient by contacting a sample of the body fluid with at least one compound which is a reducible dye, such as a tetrazolium-based compound, under conditions which allow reduction of the reducible dye. It has been found that the urine of patients suffering from a variety of diseases contains (levels of) metabolites not found in urine of healthy controls. These metabolites are particularly sulfur-containing metabolites, most particularly H₂S metabolites.

Detection of these metabolites can be used to detect, diagnose and/or monitor the presence, evolution and/or severity of diseases. The methods of the present invention can thus be used for detecting, diagnosing and/or monitoring the aberrant presence of metabolites per se or as a parameter for a disease state such as, but not limited to a disease state selected from inflammation, infectious diseases, chronic immune diseases and diseases with unknown etiology such as ME, fibromyalgia and the like. Detection of these metabolites can be used to determine or diagnose the presence of a disease state and/or monitor the evolution and/or severity of a disease state or a particular disease, e.g. to monitor the efficacy of treatment etc. More particularly, for diseases which are "silent", i.e. which do not show clearly recognizable symptoms, the methods can be used to identify a physiological problem which may need medical attention. Finally, for patients suffering from symptoms which are undefined or are not necessarily physiological, such methods can be used to demonstrate the presence of a physiological symptom.

The methods of the present invention are based on the detection of metabolites, more particularly sulfur-containing metabolites, most particularly H₂S metabolites, with a compound that us a tetrazolium dye which can be reduced by a thiol compound, whereby reduction is associated with a color change of the compound. Such compounds are also referred to herein as "reducible dyes". More particularly, such a compound is a non-toxic tetrazolium-based compound, also referred to as a tetrazolium dye, such as a tetrazolium-based salt. Examples of suitable tetrazolium-based compounds include but are not limited to 2-(2'benzothiazolyl)-5-styryl-3-(4'-phthalhydrazidyl) tetrazolium (BSPT), 2, 3, 5-triphenyl tetrazolium chloride (TTC), 2-benzothiazolyl-(2)-3,5-diphenyl tetrazolium (BTDP), 2,3-di(4-nitrophenyl) tetrazolium (DNP), 2,5-diphenyl-3-(4-styrylphenyl) tetrazolium (DPSP), distyryl nitroblue tetrazolium (DS-NBT), 3,3'-[3,3'-dimethoxy-(1,1'-biphenyl)-4,4'-diyl]-bis[2-(4-nitrophenyl)-5-phenyl(-2H tetrazolium (NBT), 3-(4,5-dimethyl-2-thiazolyl)-2,5-diphenyl-2H tetrazolium (MTT), 2-phenyl-3-(4-carboxyphenyl)-5-methyl tetrazolium (PCPM), tetrazolium blue (TB), thiocarbamyl nitroblue tetrazolium (TCNBT), tetranitroblue tetrazolium (TNBT), tetrazolium violet, (TV), 2-benzothiazothiazolyl-3-(4-carboxy-2-methoxyphenyl)-5-[4-(2-sulfoethylcar bamoyl)phenyl]-2H-tetrazolium (WST-4), and 2,2'-dibenzothiazolyl-5,5'-bis[4-di(2-sulfoethyl)carbamoylphenyl]-3,3'-(3, 3'-dimethoxy- 4,4'-biphenylene) ditetrazolium, disodium salt (WST-5). More particularly, the tetrazolium-based compound is selected from the group consisting of MTT, XTT, INT, MTS and NBT.

The reducible dyes used in the context of the present invention are typically light-colored or colorless compounds that undergo a reduction reaction, in the presence of a reducing agent, to yield a highly colored compound, such as, in the case of tetrazolium-based compounds, a highly colored formazan. Typically the color reaction is gradual and quantitative in that the intensity of the colored reaction product is dependent on the amount of reducing agent present. Accordingly, the measure of the color change is indicative of the amount of reducing agent present in the sample. For instance, NBT in unreduced form is yellow in solution and in reduced form is dark blue. It is noted that, in the presence of other colored compounds which may be present in the body fluid to be tested, such as urochrome of urine, the color of the dyes both in unreduced form and in reduced form, may be modified. For instance, shades of blue (e.g. in the reduction of NBT) will typically be observed as shades of green in the presence of urochrome. Accordingly, the color change referred to herein typically relates to the change in the color of the color reagent upon contacting with a test sample compared to a control sample (i.e. color reagent in the presence of body fluid sample not containing metabolites). Methods according to the present invention typically comprise contacting a body sample such as a urine sample with one or more reducible dyes or more particularly tetrazolium-based compounds, such as those described above, under conditions which allow reduction of said one or more dyes or compounds and observation of the color formation.

Particular embodiments of the invention accordingly provide in vitro methods, which comprise:
(i) obtaining a color reagent by dissolving at least one reducible dye such as a tetrazolium-based compound in a buffer solution, wherein the pH-value of the buffer solution is higher than the pKa- value of the reducible dye,
(ii) contacting a sample of the body fluid with the color reagent obtained in step (i), and
(iii) determining the color of said color reagent after said contacting step, whereby the color is indicative of the presence of (increased) metabolites in said sample.

Reduction of a reducible dye by a thiol compound will typically occur only at a pH which is above the pKa value of the dye. For tetrazolium-based compounds this implies that the dye should be contacted with the sample at a pH of between 5 and 11, depending on the tetrazolium-based compound used. For instance, for NBT, typically the pH should be above 8.5. Typically the suitable pH is obtained by addition of a buffer. More particularly, the dye can be dissolved in a buffer with suitable pH and then brought into contact with the sample. Alternatively, dye, buffer and sample are contacted simultaneously. Thus, where reference is made herein to a buffer with a pKa above the pKa of the reducible dye, it is intended to refer to the fact that addition of the buffer (either in dry form or in solution) to the dye will ensure a pH of the solution which is above the pKa of the reducible dye.

Different chemical compounds can be used as buffers and/or to make buffer solutions and are well known to the person skilled in the art. Typically sodium phosphate and potassium phosphate are used. Examples include, but are not limited to solutions of 10mM sodium or potassium phosphate. The buffer may contain or be mixed with other components which improve stability of the reaction product.

Accordingly, suitable buffers for use in the methods according to the present invention have a buffering capacity between a pH of between 5 and 11. Non-limiting examples of such buffers are Tris buffer, phosphate buffered saline and diethanolamine buffer and combinations thereof. In particular embodiments, a mixture of buffers is used, such as for example a mixture of phosphate buffer and Tris buffer is used. In more specific embodiments, the tetrazolium-based compounds are dissolved in a mixture of 2 volumes of phosphate buffered saline (100mM) and 1 volume of Tris buffer (100 mM) at a concentration between 1 and 20 mg/mL, such as between 1 and 10 mg/mL, wherein the resulting solution has a pH value between 8,0 and 9,0.

In the methods of the present invention, the reduction of the reducible dye, (the tetrazolium-based compound(s)), occurs by direct reaction of the sample of body fluid with the color reagent comprising at least one reducible dye (a tetrazolium-based compound) under buffered conditions. Accordingly, the reduction reaction of the dye is ensured by metabolites present in the sample. No reducing agents need to be added and the metabolites need not be isolated from the sample. Thus, contrary to most prior art assays based on the detection of tetrazolium-based compounds, where the reduction of the tetrazolium based compounds is ensured by the addition of an enzyme (e.g. used as a label for an analyte-specific reagent), in the methods of the present invention, the reducible dye is contacted directly with the sample of body fluid and is reduced by metabolites in the sample.

Accordingly, in particular embodiments, the methods according to the present invention do not make use of a combination of reagents, which for example comprises a tetrazolium-based reagent, a flavin-dependent enzyme bound to flavin, an electron transfer agent, and a nitrite salt or for example comprises tetrazolium-based reagent, an aluminum compound, a phenazine electron transfer agent and a flavin agent.

The methods of the present invention are based on the detection of an unusual production of metabolites by the body when in a diseased state compared to healthy controls, which metabolites are excreted in body fluids. Most particularly the body fluid is urine. More particularly the body fluid is whole body fluid, such as whole urine (see below). Other suitable body fluids include sweat, sputum, prostate fluid, lumbal fluid, vaginal secretion, and serum. The methods of the present invention however are of particular interest in the context of home-monitoring and self-testing. Accordingly, in particular embodiments of the invention, the body fluid is a body fluid which is a readily self-collectable body fluid, such as urine or saliva (sputum).

The sample of body fluid for use in the methods of the present invention is typically a fresh sample of body fluid. More particularly the methods are suitable for use on freshly collected urine, more particularly on a morning urine sample. Typically, the body fluid sample as such ('whole') is used directly in the methods of the invention (with the exception of blood, where the use of serum is preferred). However, under particular circumstances it may be desirable to make use of a pre-treated sample, such as for example but not limited to, a sample of body fluid from which certain molecules, compounds, cells, particles or any other material that could interfere with the reduction reaction of the metabolites with the reducible dye, herein generally referred to as "interfering factors" are removed. Methods for removing interfering factors from body fluids such as urine are known to the skilled person and include, but are not limited to sedimentation, filtration, chromatography etc. In particular embodiments, a sedimentation step is performed to remove white blood cells from the body fluid sample to be used in the methods of the invention. In some cases, presence of cells may influence the outcome of the assay. However, in most body fluids (except blood), the number of cells is relatively limited such that removal of the cells is not necessary. If necessary, the amount of color reagent used can be adjusted to ensure that presence of cells does not affect the outcome of the assay (i.e. adjusting the sensitivity of the assay). The methods of the present invention are particularly directed at detection of metabolites in "fluid".

In particular embodiments the methods of the invention are used on a sample of body fluid obtained from a mammal, more particularly from a human.

As will be detailed below, the methods of the present invention can be carried out either in solution or on a solid carrier. In particular embodiments, the methods are carried out in solution in a reaction vial, typically in volumes of 50µl-2000µl. In these embodiments, the methods of the invention comprise contacting a suitable volume of collected body fluid sample with the reducible dye and buffer solution, which is optionally pre-mixed as a "color reagent". The relative amounts of the different reagents to be combined can be determined by the skilled person using positive and negative control samples (see below). In particular embodiments, the methods of the invention comprise contacting 2 volumes of body fluid such as urine with 1 volume of color reagent (e.g. tetrazolium-based compound dissolved in appropriate buffer).

The methods of the present invention are based on the determination of the color of a reducible dye, which color is indicative of the presence of metabolites in a sample. After contacting the sample of body fluid with the color reagent comprising at least one reducible dye, the color of the color reagent is determined (i.e. where the method is carried out in solution, this may imply determining the color of the reaction solution). Determining the color of the resulting color reagent can for example be performed by comparing it with a reference. Such a reference can for example be the color of a control. For instance a negative control can be obtained by reacting a sample of the same body fluid of a healthy individual with the reducible dye (under the same conditions as those used for the test sample). Additionally or alternatively, positive controls can be obtained by reacting a sample of the same body fluid of a patient diagnosed with a particular disease with the reducible dye (under the same conditions as used for the test sample) or by spiking a sample of a healthy individual with metabolites. Additionally or alternatively, the color of the resulting reagent can be compared with the colors of a color chart or a color code, assigning a particular color to a healthy state or a disease state. Such a color chart or color code can also comprise a series of shades, wherein the different shades indicate different levels of severity of the disease. In further particular embodiments the color of the color reagent is determined spectrophotometrically. It will be understood by the skilled person that where detection of color is not possible, conversion into e.g. scales of gray of the color reaction is also possible.

The methods of the invention typically involve detecting the color associated with the reduction of the reducible dye, as a measure for the presence of (increased amounts) of metabolites in a body fluid. Contrary to existing blood tests where a tetrazolium compound is used to determine reduced white blood cell activity (and the detection or diagnosis is based on a "decrease" of reduced color reagent compared to controls), the methods of the present invention make use of reducible dyes such as tetrazolium-based compounds as an indicator of increase in metabolites, more particularly sulfur-containing metabolites, whereby detection or diagnosis is based on an "increase" of reduced color reagent.

A further aspect of the present invention provides tools and kits for carrying out the methods of the present invention.

In particular embodiments, kits are provided comprising at least one reducible dye such as a tetrazolium-based compound, a suitable buffer, a vial for collecting a body fluid, a pipetting device and a reaction container. As detailed below, the term reaction container encompasses both fluid reaction containers and carriers.

The kits and tools of the present invention are based on the detection of metabolites with a reducible dye, more particularly a tetrazolium-based compound which can be reduced by a thiol compound, whereby reduction is associated with a color change of the compound.

Different kits and tools are envisaged which are suitable for carrying out the methods of the present invention, and the physical format in which the reagents are presented are not critical to the invention. According to particular embodiments, the kits comprise a color reagent comprising the at least one reducible dye.

For use in the methods and kits of the present invention, the reducible dye may be in any form that is suitable for undergoing a reduction reaction with metabolites by directly contacting the dye with a sample of a body fluid. For example, the dye may be present as either a wet or a dry composition. By wet composition is meant a fluid composition, typically an aqueous composition, such as a buffer solution as described herein. By dry compositions is meant a composition that is not fluid, i.e., in dry form, such as a composition that is substantially free of uncombined water. Such a dry form may comprise or may not comprise constituents of a buffer, which, upon contacting with water or another solution, can be dissolved therein (e.g. powdered buffer solution).

Accordingly, in particular embodiments of the kits of the present invention, the at least one reducible dye may be dissolved in the buffer solution so as to provide a ready-to use solution that can be directly contacted with the sample of body fluid. Alternatively, the reducible dye and/or the buffer or the color reagent may be provided in powder form. Independently thereof, the dye and buffer may thus be provided in separate modules or compartments for mixing prior to usage, i.e. prior to contacting with the urine sample, or may be provided as one reagent.

In particular embodiments, the kits according to the present invention may further comprise a vial for collecting a body fluid and a pipetting device. In more particular embodiments kits are provided for urine testing which comprise a vial for collecting urine. After a sample is collected in the provided vial, the pipetting device can be used to add a suitable volume of the body fluid sample directly to the reaction container comprising one or more other reagents of the kit. Examples of suitable collection vials are disposable canisters with a cap and are known to the skilled person. Similarly, suitable pipetting devices are typically disposable (e.g. plastic) pipettes for volumes of 50-2000µl and are known to the skilled person. Where the volume of collectable body fluid is limited (e.g. sweat or saliva), the collection vial may also be a device particularly suited for collecting the body fluid (e.g. optionally comprising swabs or other devices). Alternative vials suitable for collecting different types of body fluids are known in the art.

In particular embodiments, kits are provided which are configured for carrying out the methods of the invention in solution, i.e. where the body fluid, such as urine, is contacted with the buffer and dye in one reaction container, which is a fluid container. In particular embodiments, kits are provided which comprise a fluid reaction container comprising therein a color reagent (i.e. a reducible dye readily dissolved in a suitable buffer), a vial for collecting a body fluid and a pipetting device. Contacting the urine sample with the color reagent is ensured by pipetting an appropriate volume of the collected urine directly into the reaction container.

Suitable reaction containers are known to the skilled person and the size and shape thereof is not critical to the invention. In particular embodiments, the reaction container is a fluid container. Typically, such a reaction container is a recipient of 50µl-2000µl, such as an eppendorf, cuvette, a multiwell plate, or any other such recipient.

In particular embodiments, kits are provided which are configured for carrying out the methods of the invention on a reagent container which is a carrier. The term carrier as used herein refers to a material through which a fluid reagent can pass. Examples of suitable materials are known to the person skilled in developing diagnostic test-kits and include bibulous or non-bibulous materials. By bibulous is meant a material that exhibits preferential retention of one or more components as would occur, for example, in materials capable of absorbing or "imbibing" one or more components, as occurs in chromatographic separations. Examples of bibulous materials include, but are not limited to: nylon, untreated forms of paper, nitrocellulose and the like which result in chromatographic separation of components contained in liquids which are passed therethrough.

Alternatively, the substrate may be non-bibulous. Non-bibulous substrates include inert porous matrices which provide a support for the various members of the signal producing system, described infra, and may have a positive charge. These matrices are generally configured to provide a location for application of a physiological sample, e.g., blood, and detection of the chromogenic product produced by the dye of the signal producing system. As such, the matrix is typically one that is permissive of aqueous fluid flow through it and provides sufficient void space for the chemical reactions of the signal producing system to take place.

In particular embodiments, the reagent container is provided in the form of a test strip. In particular, the invention encompasses (dry) strips for assaying metabolites in body fluids according to the methods of the invention. In the broadest sense, the reagent test strip includes a solid support and typically a dry dye, more particularly a dry tetrazolium-based compound present thereon. The dye is either coated on or associated with the material of the reagent container.

In particular embodiments, the dry dye is provided in the form of a dry color reagent, which is made up of all of the compounds necessary to produce a detectable signal in the presence of the metabolite(s). More particularly, the test strip comprises a dry color reagent comprising e.g. a tetrazolium-based compound and a buffer having a pH-value higher than the pKa value of the tetrazolium-based reagent, described in greater detail above. Additionally or alternatively, the test strip comprises only the dye and the dye is contacted with the buffer prior to adding the sample or the urine sample is mixed with a suitable buffer solution prior to contacting with the test strip. Thus, kits according to these embodiments may or may not include the buffer separately.

The test strips are envisaged to be configured in a similar way to the test strips known for other diagnostic uses. More particularly, the test strip may comprise a detection zone comprising the dye (or color reagent) which is distal on the strip and a sample application zone proximally on the strip whereby upon application of the sample of body fluid in the application zone, the urine will be moved (typically by capillary forces) through the strip material and will reach the detection zone where metabolites present in the sample will react with the color reagent.

In particular embodiments the reagent container may be in the form of a carrier that is affixed to a solid support. The support may be a plastic, e.g. polystyrene, nylon, or polyester, or metallic sheet or any other suitable material known in the art. The strip may also be configured in more complex arrangements, e.g., where the test pad is present between the support and a surface layer, where one or more reagents employed in sample processing may be present on the surface layer. In addition, flow paths or channels may be present on the test strip, as is known in the art.

Alternatively the reaction container may be in the form of a carrier provided in a container, such as a porous matrix in a longitudinal vial (e.g. column). Typically, in these embodiments a sample will flow vertically through the carrier as a result of gravity or by application of a force.

The dimensions and porosity of the material of the carrier may vary greatly, where the matrix may or may not have a porosity gradient, e.g., with larger pores near or at the sample application region and smaller pores at the detection region. The matrix may be configured as a membrane test pad and be affixed to a solid support, where the support may be a plastic (e.g., polystyrene, nylon or polyester) or metallic sheet or any other suitable material known in the art.

The carriers may be fabricated employing any convenient protocol. One convenient protocol is to contact at least the test pad portion of the strip with an aqueous composition that includes the reducible dye and a buffer solution having a pH value that is higher than the pKa value of the reducible dye. Conveniently, the test pad may be immersed in the aqueous composition, maintained therein for a sufficient period of time and then dried, whereby the test pad of the reagent test strip which has associated therewith the reagent composition is produced.

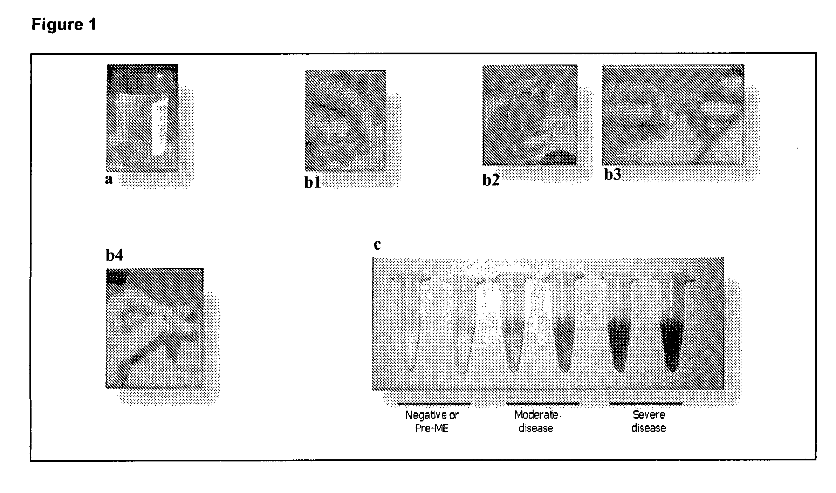

In particular embodiments, the kits provided according to the invention further provide tools for interpreting the obtained result, i.e. the color reaction observed in the reaction container. Indeed, the reagents of kits of the invention are configured such that the color observed in the reaction container is indicative of the presence of metabolites in the sample. Determining the color of the resulting color reagent (and/or interpreting the result of the assay) can for example be performed by comparing the observed color with a reference. Accordingly, the kits according to the present invention may further comprise a color reference. Such a color reference can for example be a color chart or a color code, typically comprising different shades corresponding to the colors obtained with different concentrations of metabolite present in the body sample. In particular embodiments references are provided assigning a particular color to a healthy state or a (particular stage of a) disease state. Such a color chart or color code can also comprise different shades of the color reaction, with an indication of how the colors are indicative of different levels of severity of disease. Such references may be provided in the form of one or more reaction containers containing different shades of the dye or may be provided on paper reproducing the different shades of the dye (see Figure 1c).

In addition to above mentioned components, the kits of the present invention may typically further include instructions for using the components of the kit to practice the methods according to the invention. The instructions for practicing the methods of the invention are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided.

Typically, the kits of the present invention do not comprise any additional reducing agents and the metabolites need not be isolated from the sample. Thus, contrary to most prior art kits based on the detection of tetrazolium-based compounds, where the reduction of the tetrazolium based compounds is ensured by the addition of an enzyme (e.g. used as a label for an analyte-specific reagent), in the kits of the present invention, the reducible dye is contacted directly with the urine sample and is reduced by metabolites in the sample.

Accordingly, the kits according to the present invention do not comprise a combination of reagents, which for example comprises a tetrazolium-based reagent, a flavin-dependent enzyme bound to flavin, an electron transfer agent, and a nitrite salt or for example comprises tetrazolium-based reagent, an aluminum compound, a phenazine electron transfer agent and a flavin agent.

In particular embodiments the invention accordingly provides kits consisting of a limited number of components which provide all the necessary tools for ensuring detection of metabolites in a body fluid. More particularly these tools are a collection vial for collecting body fluid, a pipette, and a reaction vial which comprises the color reagent dissolved in the appropriate buffer and a packaging insert comprising details on the interpretation of the color reaction. Such kits are particularly suitable for home-testing or testing in a medical practitioner's office.

The methods and kits according to the present invention may be (in vitro) diagnostic methods and diagnostic kits that can be used for detecting, diagnosing and/or monitoring aberrant production of metabolites in a patient, which are indicative of a disease state. In particular, the methods and kits of the present invention allow detecting the presence of metabolites that are produced in the intestinal tract of patients suffering from inflammatory, infectious and/or chronic immune diseases, such as infectious diseases (including viral, bacterial, prion-related infectious diseases), cancers, mental disorders, autism, immunologic disorders (including auto-immune diseases, rheumatoid arthritis and the like), nervous system disorders, disorders of the gut, defective wound healing processes, intoxications (with e.g. heavy metals, pesticides and the like), unexplained syndromes, aging related processes, biological clock related processes and diseases with unknown etiology such as CFS/ME, fibromyalgia and the like. Thus the methods and kits of the present invention can be used to detect the presence of a "disease state" (which may be, but is not limited to any one of the disease states listed above) or to monitor the severity of a previously identified disease state.

In particular embodiments, the methods and kits of the present invention can be used for the detection, diagnosis and monitoring of CFS/ME. It has been demonstrated that patients suffering from CFS/ME are characterized by an increased H₂S metabolism, correlating with the intensity of the symptoms. This is reflected in the urine of such patients. The symptoms of CFS/ME are however not easily recognized or accepted as "physiological". The methods and kits of the present invention for detecting, diagnosing and/or monitoring diseases based on the detection of metabolites, more particularly sulfur-containing metabolites, most particularly H₂S metabolites, are useful to demonstrate a truly physiological symptom, as an indicator of these diseases.

Finally, the methods and kits of the present invention are useful in alerting patients to seek medical advice and/or in assisting medical staff in recognizing a disease state where a disease may be "silent" and difficult to diagnose.

The methods and kits according to the present invention are broad-spectrum detection tools. These may be combined with one or more additional assays or test kit (components) to further identify the nature of the disease state.

For instance the methods and kits for determining a disease state based on the presence of metabolites in a body fluid may be combined with an assay which determines e.g. the presence of heavy metals in a body fluid sample, which is known to be indicative for CFS/ME. Alternatively and for example, the methods and kits of the present invention may be combined with a test for measuring the presence of organophosphorus ompounds in the urine sample, which are known to be indicative for chronic immune diseases.

The present invention is further illustrated by the following non-limiting figure and examples:

### FIGURE LEGEND

**Fig. 1** **Illustration of one of the embodiments of the methods according to the**

### invention

a) Step 1: Urine is collected in a vial
b1) to b4) Step 2: Urine sample is contacted with the color reagent and mixed, and allowed to react for two minutes
c) Step 3: observe color change. Dark color = positive sample
The color of the color reagent is optionally determined using a color chart, whereby the color is indicative the severity of a physiological disease.

### EXAMPLES

### Example 1: Correlation of MTT reduction by urine with disease severity

In an eppendorf, 200 µl solution of colorimetric reagent was contacted with 400 µl morning urine obtained from different patients with varying symptoms causing mild to significant discomfort. The reaction vial is mixed and after two minutes the color change is observed (method illustrated in Figure 1).

In Figure 1 c) a colour chart is shown, indicating the range of colours that is observed with MTT as the colorimetric probe contacted with urine samples of patients having different disease states.

The following correlation between coloration and disease state could be determined:
0: yellow - healthy control
+/- dark yellow - one needs to be alert
+ light green - medical consultation required
++ dark brown - medical consultation required
+++ light purple - urgent medical consultation required
++++ deep purple - urgent medical consultation required

In the same way, follow up on therapy on successful therapy, starting from a strong positive case ( purple colour +++) should lead over a brown colour ( positive++ ) back to the yellow colour ( 0 ).

### Example 2: blind testing of samples of body fluids with the methods according to the invention

In order to confirm the reliability of the methods of the invention for determining the presence and/or severity of a disease condition, a set of 40 samples of patients which were independently diagnosed based on other symptoms was tested. The results were as follows:

### Test results of urine samples obtained from patients with the following diagnosis :

| NR | OD value | Score | Diagnosis |
|---|---|---|---|
| 1 | 3.200 | ++++ | ME G93.3, fructose intolerance, HHV6 |
| 2 | 1.111 | ++++ | ME, Borreliosis Food Intolerance |
| 3 | 0.544 | +++ | Has had ME, almost well, food intolerance? low NK cells |
| 4 | 0.363 | ++ | Food intolerance, low NK, fructose malabsorption, HHV6 |
| 5 | 0.137 | + | Fatigue, low NK cells |
| 6 | | | EBV, fructose malabsorption, lactose intolerance |
| 7 | 0.607 | +++ | ME. Borreliosis? |
| 8 | 0.733 | +++ | ME Bartonella? Fructose malabsorption, lactose intolerance |
| 9 | 0.123 | + | Burned out, atopic exema |
| 10 | 0394 | ++ | ME |
| 11 | 0.094 | + /- | ADHD |
| 12 | 0.296 | ++ | ME |
| 13 | 0.065 | - | Gamma GT=100-300 last 6 years |
| 14 | 0.298 | ++ | ME |
| 15 | 0.090 | +/- | ADHD |
| 16 | 0.393 | ++ | Possible ADHD? Fatigue, unstable moods, sweaty smell |
| 17 | 0.898 | +++ | Artrose, Migraine |
| 18 | 0.116 | + | ADHD |
| 19 | 0.325 | ++ | ME |
| 20 | 0.119 | + | Milk intolerance, pale, anemia, sometimes fatigued |
| 21 | 0.108 | + | Food intolerance, fatigue |
| 22 | 0.883 | +++ | Has all symptoms of ME, GP thinks its ME |
| 23 | 0.275 | ++ | ME |
| 24 | 0.093 | +/- | MS, Diabetes, Epilepsy |
| 25 | 0.958 | +++ | Severe ME |
| 26 | 0.093 | +/- | Migrene, diagnosed "burn out" 7 years ago |
| 27 | 2.378 | ++++ | ME |
| 28 | 0.120 | + | MS |
| 29 | 0.846 | +++ | ME moderate to severe |
| 30 | 0.318 | ++ | ME |
| 31 | 0.103 | + | Fybromyalgia |
| 32 | 0.265 | ++ | Bechterew, osteopeny, allergy, struma |
| 33 | 0.525 | +++ | ADHD/ADD?? currarino syndrome, dysleksi , chronic urinary infection, still fatigued, sleeps a lot |
| 34 | 0.528 | +++ | ME |
| 35 | 0.325 | ++ | ME |
| 36 | 1.049 | +++ | ME/ Fibriomyalgia |
| 37 | 0.205 | ++ | Epilepsy, ADHD, migraine, insomnia, cramps, muscle pain, dyslecsia |
| 38 | 0.285 | ++ | Migraine, many infections, fatigue, insomnia, muscle pain, cramps |
| 39 | 0.030 | - | Healthy control |
| 40 | 0.080 | - | Healthy control |

It is observed that the score in the urinary aanalysis correlates well with disease state. In patients with ME, the score is at least ++.

## Claims

1. An in vitro method for detecting or monitoring a disease state in a patient, said method comprising detecting the presence of metabolites in urine by contacting a urine sample of said patient with at least one tetrazolium dye at a pH-value which is higher than the pKa-value of said tetrazolium dye and between 5 and 11 under buffered conditions without adding any additional reducing agents, whereby a reduction of the tetrazolium dye occurs by direct reaction of said metabolites in the urine with said at least one tetrazolium dye and whereby a color change resulting from reduction of the tetrazolium dye is indicative of the increased presence of metabolites in the urine and thus indicative of a disease state in said patient.

2. The in vitro method of claim 1, wherein said disease state is selected from the group consisting of infectious diseases, cancers, mental disorders, autism, immunologic disorders, nervous system disorders, disorders of the gut, defective wound healing processes, intoxications, unexplained syndromes, ageing related processes, biological clock related processes and diseases with unknown etiology.

3. The in vitro method of claim 1 or 2, which comprises:
(i) obtaining a color reagent by dissolving said at least one tetrazolium dye in a buffer solution, wherein the pH-value of the buffer solution is between 5 and 11 and higher than the pKa- value of the tetrazolium dye,
(ii) contacting the sample with the color reagent obtained in step (i) without adding any additional reducing agents, whereby a reduction of the tetrazolium dye occurs by direct reaction of said metabolites in the urine with said at least one tetrazolium dye and
(iii) determining the color of said color reagent after said contacting step (ii), whereby the color is indicative of the increased presence of metabolites in said urine sample and indicative of a disease state.

4. The in vitro method of claim 3, wherein step (iii) comprises comparing the color of the color reagent with a reference.

5. The in vitro method of any of claims 1 to 4, which method is used to compare the production of said metabolites in the body of said patient compared to healthy controls.

6. The in vitro method of any of claims 1 to 5, which is a method for detecting, diagnosing and/or monitoring a disease related to inflammatory, infectious and/or chronic immune disease.

7. The in vitro method of claim 1 or 3 to 6, wherein said disease is Chronic Fatigue Syndrome/Myalgic Encephalomyelitis (CFS/ME).

8. The in vitro method of any of claims 1 to 7, wherein the at least one tetrazolium dye is chosen from the group consisting of 3-(4.5-dimethyl-2-thiazolyl)-2,5-diphenyl-2H tetrazolium (MTT), 2,3-bis-(2-methoxy-4-nitro-5-sulfophenyl)-2H-tetrazolum -5-carboxnilide (XTT), 2-(4-iodophenyl)-3-(4-nitrophenyl)-5-phenyl tetrazolium (INT), 3-(4,5-dimethylthiazol-2-yl)-5-(3-carboxymethoxyphenyl)-2-(4-sulfophenyl)-2H-tetrazolium (MTS) and 3,3'-[3,3'-dimethoxy-(1,1'-biphenyl)-4,4'-diyl]-bis[2-(4-nitrophenyl)-5-phenyl-(2H tetrazolium (NBT).

9. The in vitro method of any one of claims 1 to 8, wherein said urine sample is a morning urine sample.

10. The in vitro method of any one of claims 1 to 9, wherein said metabolites are thiols.

11. Use of a kit comprising at least one tetrazolium dye, a buffer with a pH between 5 and 11 which is higher than the pKa value of the tetrazolium dye, a reaction container, a vial for collecting urine, and a pipetting device, wherein said kit does not comprise any additional reducing agents for detecting, diagnosing and/or monitoring a disease state of a patient by detecting increased metabolite production in a urine sample of said patient.

12. The use of claim 11, which comprises diagnosing and/or monitoring a disease related to inflammatory, infectious and/or chronic immune disease.

13. The use of claim 11 or 12, which comprises the detection, diagnosis and monitoring of Chronic Fatigue Syndrome.

14. The use of any one of claims 11 to 13, wherein the at least one reducible tetrazolium dye is chosen from the group consisting of 3-(4.5-dimethyl-2-thiazolyl)-2,5-diphenyl-2H tetrazolium (MTT), 2,3-bis-(2-methoxy-4-nitro-5-sulfophenyl)-2H-tetrazolum -5-carboxnilide (XTT), 2-(4-iodophenyl)-3-(4-nitrophenyl)-5-phenyl tetrazolium (INT), 3-(4,5-dimethylthiazol-2-yl)-5-(3-carboxymethoxyphenyl)-2-(4-sulfophenyl)-2H-tetrazolium (MTS) and 3,3'-[3,3'-dimethoxy-(1,1'-biphenyl)-4,4'-diyl]-bis[2-(4-nitrophenyl)-5-phenyl-(2H tetrazolium (NBT).

## Patentansprüche

1. In-vitro-Verfahren zum Feststellen oder Überwachen eines Krankheitszustands in einem Patienten, wobei das Verfahren das Feststellen des Vorhandenseins von Metaboliten im Urin durch Inkontaktbringen einer Urinprobe des Patienten mit mindestens einem Tetrazoliumfarbstoff bei einem pH-Wert umfasst, der höher ist als der pH-Wert des Tetrazoliumfarbstoffs und unter gepufferten Bedingungen zwischen 5 und 11 liegt, ohne Zugabe zusätzliche Reduktionsmittel, wobei eine Reduktion des Tetrazoliumfarbstoffs durch direkte Reaktion der Metaboliten in dem Urin mit dem mindestens einem Tetrazoliumfarbstoff stattfindet und wobei eine Farbveränderung als Resultat der Reduktion des Tetrazoliumfarbstoffs das erhöhte Vorhandensein von Metaboliten in dem Urin anzeigt und damit einen Krankheitszustand in dem Patienten anzeigt.

2. In-vitro-Verfahren nach Anspruch 1, wobei der Krankheitszustand aus der Gruppe ausgewählt ist, die aus Infektionskrankheiten, Krebskrankheiten, Geistesstörungen, Autismus, immunologischen Störungen, Störungen des Nervensystems, Störungen des Darms, defekten Wundheilungsprozessen, Vergiftungen, unerklärlichen Symptomen, altersbedingten Prozessen, mit der biologische Uhr in Zusammenhang stehenden Prozessen und Krankheiten mit unbekannter Ätiologie besteht.

3. In-vitro-Verfahren nach Anspruch 1 oder 2, welches Folgendes umfasst:
(i) Erhalten eines Farbreagenz durch Lösen des mindestens einen Tetrazoliumfarbstoffs in einer Pufferlösung, wobei der pH-Wert der Pufferlösung zwischen 5 und 11 liegt und höher ist als der pKa-Wert des Tetrazoliumfarbstoffs,
(ii) Inkontaktbringen des in Schritt (i) Farbreagenz ohne Zugabe zusätzlicher Reduktionsmittel, wobei eine Reduktion des Tetrazoliumfarbstoffs durch direkte Reaktion der Metaboliten in dem Urin mit dem mindestens einem Tetrazoliumfarbstoff stattfindet, und
(iii) Bestimmen der Farbe des Farbreagenz nach dem Schritt des Inkontaktbringens (ii), wobei die Farbe das erhöhte Vorhandensein von Metaboliten in der Urinprobe anzeigt und damit einen Krankheitszustand anzeigt.

4. In-vitro-Verfahren nach Anspruch 3, wobei Schritt (iii) das Vergleichen der Farbe des Farbreagenz mit einer Referenz umfasst.

5. In-vitro-Verfahren nach einem der Ansprüche 1 bis 4, wobei das Verfahren verwendet wird, um die Produktion der Metaboliten im Körper des Patienten gegenüber gesunden Kontrollen zu vergleichen.

6. In-vitro-Verfahren nach einem der Ansprüche 1 bis 5, wobei es sich um ein Verfahren zum Feststellen, Diagnostizieren und/oder Überwachen einer Krankheit in Zusammenhang mit einer entzündlichen, infektiösen und/oder chronischen Immunkrankheit handelt.

7. In-vitro-Verfahren nach Anspruch 1 oder 3 bis 6, wobei es sich bei der Krankheit um chronisches Erschöpfungssyndrom/myalgische Enzephalomyelitis (CFS/ME) handelt.

8. In-vitro-Verfahren nach einem der Ansprüche 1 bis 7, wobei der mindestens eine Tetrazoliumfarbstoff aus der Gruppe ausgewählt ist, die aus 3-(4,5-Dimethyl-2-thiazolyl)-2,5-diphenyl-2H-tetrazolium (MTT), 2,3-Bis-(2-methoxy-4-nitro-5-sulfophenyl)-2H-tetrazolium-5-carbox-anilid (XTT), 2-(4-lodphenyl)-3-(4-nitrophenyl) -5-phenyltetrazolium (INT), 3(4,5-Dimethylthiazol-2-yl)-5-(3-carboxymethoxyphenyl)-2-(4-sulfophenyl)-2H-tetrazolium (MTS) und 3,3'-[3,3'-Dimethoxy-(1,1'-biphenyl)-4,4'-diyl]-bis[2-(4-nitrophenyl) - 5-phenyl-(2H-tetrazolium (NBT) besteht.

9. In-vitro-Verfahren nach einem der Ansprüche 1 bis 8, wobei es sich bei der Urinprobe um eine Morgenurinprobe handelt.

10. In-vitro-Verfahren nach einem der Ansprüche 1 bis 9, wobei es sich bei den Metaboliten um Thiole handelt.

11. Verwendung eines Kits, umfassend mindestens einen Tetrazoliumfarbstoff, einen Puffer mit einem pH-Wert zwischen 5 und 11, der höher ist als der pKa-Wert des Tetrazoliumfarbstoffs, einen Reaktionsbehälter, ein Gefäß zum Sammeln von Urin und eine Pipettiervorrichtung, wobei das Kit keine zusätzlichen Reduktionsmittel umfasst, zum Feststellen, Diagnostizieren und/oder Überwachen eines Krankheitszustands eines Patienten durch Feststellen einer erhöhten Metabolitproduktion in einer Urinprobe des Patienten.

12. Verwendung nach Anspruch 11, welche das Diagnostizieren und/oder Überwachen einer Krankheit in Zusammenhang mit einer entzündlichen, infektiösen und/oder chronischen Immunkrankheit umfasst.

13. Verwendung nach Anspruch 11 oder 12, welches die Feststellung, Diagnose und Überwachung von chronischem Erschöpfungssyndrom umfasst.

14. Verwendung nach einem der Ansprüche 11 bis 13, wobei mindestens ein reduzierbarer Tetrazoliumfarbstoff aus der Gruppe ausgewählt ist, die aus 3-(4,5-Dimethyl-2-thiazolyl)-2,5-diphenyl-2H-tetrazolium (MTT), 2,3-Bis-(2-methoxy-4-nitro-5-sulfophenyl)-2H-tetrazolum-5-carbox-anilid (XTT), 2-(4-lodphenyl)-3-(4-nitrophenyl)-5-phenyltetrazolium (INT), 3(4,5-Dimethylthiazol-2-yl)-5-(3-carboxy-methoxyphenyl)-2-(4-sulfophenyl)-2H-tetrazolium (MTS) und 3,3'-[3,3'-Dimethoxy-(1,1'-biphenyl)-4,4'-diyl]-bis[2-(4-nitrophenyl)-5-phenyl-(2H-tetrazolium (NBT) besteht.

## Revendications

1. Procédé *in vitro* de détection ou de surveillance d'un état pathologique chez un patient, ledit procédé comprenant la détection de la présence de métabolites dans l'urine par mise en contact d'un échantillon d'urine dudit patient avec au moins un colorant tétrazolium à une valeur de pH qui est supérieure à la valeur de pKa dudit colorant tétrazolium et entre 5 et 11 dans des conditions tamponnées sans ajout d'agents de réduction supplémentaires, une réduction du colorant tétrazolium se produisant ainsi par réaction directe desdits métabolites dans l'urine avec ledit ou lesdits colorants tétrazolium et un changement de couleur résultant de la réduction du colorant tétrazolium indiquant ainsi la présence accrue de métabolites dans l'urine et indiquant par conséquent un état pathologique chez ledit patient.

2. Procédé *in vitro* selon la revendication 1, dans lequel ledit état pathologique est sélectionné dans le groupe constitué des maladies infectieuses, des cancers, des troubles mentaux, de l'autisme, des troubles immunologiques, des troubles du système nerveux, des troubles intestinaux, des processus de cicatrisation défectueux, des intoxications, des syndromes inexpliqués, des processus liés au vieillissement, des processus liés à l'horloge biologique et des maladies d'étiologie inconnue.

3. Procédé *in vitro* selon la revendication 1 ou 2, qui comprend :
(i) l'obtention d'un réactif coloré par dissolution dudit ou desdits colorants tétrazolium dans une solution tampon, dans lequel la valeur de pH de la solution tampon est comprise entre 5 et 11 et est supérieure à la valeur de pKa du colorant tétrazolium,
(ii) la mise en contact de l'échantillon avec le réactif coloré obtenu dans l'étape (i) sans ajouter d'agents de réduction supplémentaires, une réduction du colorant tétrazolium se produisant ainsi par réaction directe desdits métabolites dans l'urine avec ledit ou lesdits colorant tétrazolium et
(iii) la détermination de la couleur dudit réactif coloré après ladite étape de mise en contact (ii), la couleur indiquant ainsi la présence accrue de métabolites dans ledit échantillon d'urine et indiquant un état pathologique.

4. Procédé *in vitro* selon la revendication 3, dans lequel l'étape (iii) comprend la comparaison de la couleur du réactif coloré à une référence.

5. Procédé *in vitro* selon l'une quelconque des revendications 1 à 4, ledit procédé étant utilisé pour comparer la production desdits métabolites dans le corps dudit patient à ladite production chez des contrôles sains.

6. Procédé *in vitro* selon l'une quelconque des revendications 1 à 5, qui est un procédé de détection, de diagnostic et/ou de surveillance d'une maladie liée à une maladie inflammatoire, infectieuse et/ou immunitaire chronique.

7. Procédé *in vitro* selon les revendications 1 ou 3 à 6, dans lequel ladite maladie est le syndrome de fatigue chronique/ encéphalomyélite myalgique (SFC/EM).

8. Procédé *in vitro* selon l'une quelconque des revendications 1 à 7, dans lequel le ou les colorants tétrazolium sont choisis dans le groupe constitué du 3-(4,5-diméthyl-2-thiazolyl)-2,5-diphényl-2H tétrazolium (MTT), du 2,3-bis-(2-méthoxy-4-nitro-5-sulfophényl)-2H-tétrazolium-5-carboxanilide (XTT), du 2-(4-iodophényl)-3-(4-nitrophényl)-5-phényl tétrazolium (INT), du 3-(4,5-diméthylthiazol-2-yl)-5-(3-carboxyméthoxyphényl)-2-(4-sulfophényl)-2H-tétrazolium (MTS) et du 3,3'-(3,3'-diméthoxy-(1,1'-byphényl)-4,4'-diyl]bis(2-(4-nitrophényl)-5-phényl-2H-tétrazolium (NBT).

9. Procédé *in vitro* selon l'une quelconque des revendications 1 à 8, dans lequel ledit échantillon d'urine est un échantillon d'urine matinale.

10. Procédé *in vitro* selon l'une quelconque des revendications 1 à 9, dans lequel lesdits métabolites sont les thiols.

11. Utilisation d'un kit comprenant au moins un colorant tétrazolium, un tampon avec un pH de 5 à 11 qui est supérieur à la valeur de pKa du colorant tétrazolium, un récipient de réaction, un flacon pour recueillir l'urine, un dispositif de pipetage, dans lequel ledit kit ne comprend pas d'agent de réduction supplémentaire pour la détection, le diagnostic et/ou la surveillance d'un état pathologique d'un patient par détection d'une production accrue de métabolites dans un échantillon d'urine dudit patient.

12. Utilisation selon la revendication 11, qui comprend le diagnostic et/ou la surveillance d'une maladie liée à une maladie inflammatoire, infectieuse et/ou immunitaire chronique.

13. Utilisation selon la revendication 11 ou 12, qui comprend la détection, le diagnostic et la surveillance du syndrome de fatigue chronique.

14. Utilisation selon l'une quelconque des revendications 11 à 13, dans lequel le ou les colorants tétrazolium réductibles sont choisis dans le groupe constitué du 3-(4,5-diméthyl-2-thiazolyl)-2,5-diphényl-2H tétrazolium (MTT), du 2,3-bis-(2-méthoxy-4-nitro-5-sulfophényl)-2H-tétrazolium-5-carboxanilide (XTT), du 2-(4-iodophényl)-3-(4-nitrophényl)-5-phényl tétrazolium (INT), du 3-(4,5-diméthyl-thiazol-2-yl)-5-(3-carboxyméthoxyphényl)-2-(4-sulfophényl)-2H-tétrazolium (MTS) et du 3,3'-(3,3'-diméthoxy-(1,1'-byphényl)-4,4'-diyl]bis(2-(4-nitrophényl)-5-phényl-2H-tétrazolium (NBT).
